# FASCICULE DE BREVET EUROPEEN

(11) **EP 0 596 788 B1**
(45) Date de publication et mention de la délivrance du brevet: **11.12.1996**
(21) Numéro de dépôt: 93402676.6
(22) Date de dépôt: 29.10.1993
(51) Int. Cl.: A61B 17/70

(54) **Dispositif d'ostéosynthèse pour consolidation rachidienne**
Osteosynthesevorrichtung zur Wirbelsäulenverfestigung
Osteosynthesis device for spinal consolidation

(30) Priorité: 06.11.1992 FR 9213414
(43) Date de publication de la demande: 11.05.1994
(73) Titulaire: BIOMAT(S.a.r.l.), F-91430 Igny (FR)
(72) Inventeur: Lahille, Michel, F-91430 Vauhallan (FR); Lemaire, Jean-Philippe, F-21560 Couternon (FR); Khalife, Sami, Clos de l'Entonoir, F-80000 Amiens (FR)
(74) Mandataire: Cabinet Martinet & Lapoux

(56) Documents cités:
- EP-A- 0 240 034
- EP-A- 0 408 489
- WO-A-91/01691
- DE-A- 2 933 637
- FR-A- 2 659 225

## Description

La présente invention concerne un dispositif d'ostéosynthèse comprenant une tige, et un élément ayant un tronçon fileté s'étendant à partir d'une tête et muni d'un écrou.

Un tel dispositif d'ostéosynthèse connu pour stabiliser et corriger le rachis est muni au moins de deux vis, en tant qu'élément à tronçon fileté. Outre le tronçon fileté servant à la relier à la tige de consolidation au moyen de l'écrou, chacune des vis comprend une partie partiellement ou complètement filetée à implanter le plus souvent dans le pédicule d'une vertèbre.

La tige de consolidation s'étend le long de l'apophyse épineuse du rachis et doit être reliée rapidement aux tronçons filetés de plusieurs vis pédiculaires, tout en assurant un positionnement précis des vis l'une par rapport à l'autre et surtout une liaison démontable efficace entre les vis et la tige.

Selon la technique antérieure, la tige est reliée à une vis en serrant la tige entre deux écrous adéquats vissés sur le tronçon fileté de vis et latéralement à ce dernier. Selon une autre variante connue, une portion de la tige est coincée entre une rondelle ayant une bordure en forme de demi-gouttière et une arête chanfreinée de l'écrou; l'écrou pousse la tige sur la rondelle et la rondelle contre la tête de vis qui sépare le tronçon fileté et la partie de vis implantée.

Ces liaisons connues entre vis et tige n'offrent pas de portée suffisante aux écrous, ou à la rondelle en demi-gouttière et l'écrou, sur la tige pour empêcher la rotation, voire la translation, de la tige. Le tronçon fileté et surtout les écrous subissent des efforts asymétriques par rapport à leur axe ce qui accroit le risque de rupture de ces pièces notamment lorsque la tige de consolidation est soumise à une flexion ou torsion du rachis.

La EP-A-0 408 489 décrit un dispositif d'ostéosynthèse remédiant aux inconvénients précédents grâce à une attache de liaison, comprenant une tige avec filetages à droite et à gauche, un élément ayant un tronçon fileté s'étendant à partir d'une tête intermédiaire et muni d'un écrou, et une attache de liaison. L'attache comprend un premier alésage traversé par ladite tige, une fente débouchant axialement dans le premier alésage, et un second alésage sensiblement perpendiculaire à la première fente et traversé par une partie dudit tronçon fileté localisée entre la tête et l'écrou.

Selon cette demande de brevet, d'une part, la tige de consolidation est filetée pour être vissée dans le premier alésage qui est taraudé. Cette liaison par filetage oblige le chirurgien à tourner la tige pour ajuster les positions de plusieurs attaches sur la tige en fonction de la position des vis pédiculaires et surtout ne permet pas de positionner d'autres attaches en face de vis lorsqu'une attache est serrée sur la tige par l'écrou correspondant.

D'autre part, ledit élément selon la EP-A-0 408 489 est une vis d'ancrage dont la tête intermédiaire bombée enserre, avec l'écrou ayant également une surface de contact bombée, les branches de l'attache partagées par la fente. Toutefois, lors de l'assemblage, les vis d'ancrage étant déjà implantées, les seconds alésages des attaches déjà vissées sur la tige de consolidation doivent être préalablement orientés et positionnés en regard respectivement des tronçons filetés des vis implantées. Ce prépositionnement est très difficile à effectuer. En outre ce prépositionnement en combinaison avec le vissage de la tige dans les attaches ne permet pas au chirurgien de régler les distances et positions relatives des attaches entr'elles et en conséquence les efforts de traction/poussée sur les tronçons de la tige de consolidation entre les attaches.

La présente invention vise à améliorer la commodité et la rapidité du montage, tout en garantissant un réglage aisé des distances et positions relatives des attaches sur la tige de consolidation.

A cette fin, un dispositif d'ostéosynthèse selon l'invention est tel que défini dans la revendication 1.

L'attache de liaison logée partiellement entre l'écrou et la tête de vis offre une surface porteuse sur la périphérie de la tige incomparablement plus élevée que les écrous connus ou la combinaison connue de l'écrou et la rondelle à demi-gouttière. Grâce au pinçage, l'attache peut enserrer quasiment totalement un tronçon de la tige et donc adhérer à la quasi-totalité de la périphérie de la section de la tige. L'assemblage est particulièrement rigide notamment contre toute tentative de rotation de la tige autour de son axe.

Le caractère lisse du premier alésage autorise un glissement de toute attache non serrée indépendamment d'autres attaches.

Lorsque la première fente avant montage a une largeur relativement faible, l'écrou serrant les branches de l'attache séparées par la fente est utilisé naturellement de sorte que l'écrou ne soit pas sollicité par des efforts proches de directions radiales.

La seconde fente peut être sensiblement perpendiculaire ou parallèle au premier alésage, afin de faciliter l'introduction de l'attache entre l'écrou et la tête d'une vis particulièrement lorsque la tige de consolidation relie de nombreuses vis et autres éléments à tronçon fileté.

Selon une autre variante de l'invention, la jupe cylindrique de l'écrou possède un diamètre qui est sensiblement égal au diamètre du second alésage et supérieur à la largeur de la seconde fente.

La seconde fente plus étroite que le diamètre de la jupe et du second alésage maintient le tronçon fileté de la vis dans l'attache de liaison lorsque l'écrou est vissé sur le tronçon fileté et retrécit la première fente.

Selon une autre réalisation, un élément à tronçon fileté peut comporter une partie en forme de griffe séparée dudit tronçon fileté par la tête.

Le dispositif peut comprendre un moyen ayant un alésage traversé à coulissement par la tige et ayant une partie en forme de griffe, et un moyen de pression radial à la tige pour maintenir immobile ledit moyen monté à coulissement sur la tige. Ces éléments et moyens coulissants en forme de griffe sont destinés à s'accrocher à des apophyses vertébraux.

L'invention concerne également la stabilisation transversale entre deux tiges de consolidation côte à côté, par exemple disposées à droite et à gauche de l'apophyse épineuse, et la stabilisation longitudinale entre deux tiges le long du même côté de l'apophyse épineuse.

Selon une autre variante de l'invention, lorsqu'une seconde tige de consolidation est disposée latéralement à la première tige précitée, un premier moyen transversal de stabilisation reliant les première et seconde tiges comprend
une plaque transversale aux tiges,
deux crochets ayant chacun un tronçon fileté traversant une lumière de la plaque et une partie de prise entourant partiellement l'une respectif des première et seconde tiges, et
deux écrous vissés respectivement autour des tronçons filetés de crochet pour serrer la plaque contre les première et seconde tiges afin d'emprisonner les tiges entre la plaque et les parties de prise de crochet.

Selon une autre variante, un moyen transversal de stabilisation comprend
une barre transversale aux tiges,
deux crochets ayant des parties de prise à crocheter aux tiges, et des moyens pour fixer rapidement les crochets à des extrémités de la barre.

Selon l'invention, un moyen longitudinal de stabilisation entre des première et troisième tige de consolidation sensiblement parallèles, par exemple entre des extrémités de tiges sensiblement en prolongement l'une de l'autre, comprend un raccord ayant un orifice longitudinal à section oblongue traversé par les première et troisième tiges, et
un moyen pour écarter les première et troisième tiges afin de bloquer les première et troisième tiges dans ledit orifice.

D'autres caractéristiques et avantages de la présente invention apparaîtront plus clairement à la lecture de la description suivante de plusieurs réalisations préférées de l'invention, en référence aux dessins annexés correspondants dans lesquels :
- la figure 1 est une vue en perspective éclatée d'un assemblage de base entre une vis pédiculaire et une tige de consolidation dans un dispositif d'ostéosynthèse selon l'invention ;
- la figure 2 est une vue en perspective d'une première attache pour relier une tige de consolidation à un élément à tronçon fileté tel que vis pédiculaire ou première griffe vertébrale ;
- la figure 3 est une vue en perspective d'une seconde attache pour relier une tige de consolidation à un élément à tronçon fileté ;
- la figure 4 est une vue en perspective éclatée d'un autre assemblage de base entre une tige de consolidation et une griffe vertébrale à tronçon fileté dans un dispositif d'ostéosynthèse selon l'invention ;
- la figure 5 est une vue en perspective partiellement éclatée d'une seconde griffe vertébrale montée à coulissement sur une tige de consolidation ;
- la figure 6 est une vue en perspective schématique d'un dispositif d'ostéosynthèse comprenant plusieurs tiges de consolidation reliées par des moyens transversaux et longitudinaux de stabilisation ;
- la figure 7 est une vue en perspective partiellement éclatée d'un premier moyen transversal de stabilisation à plaque crantée ;
- la figure 8 est une vue en perspective éclatée d'un second moyen transversal de stabilisation à barre filetée ; et
- la figure 9 est une vue transversale en bout d'un raccord longitudinal de stabilisation pour deux tiges de consolidation.

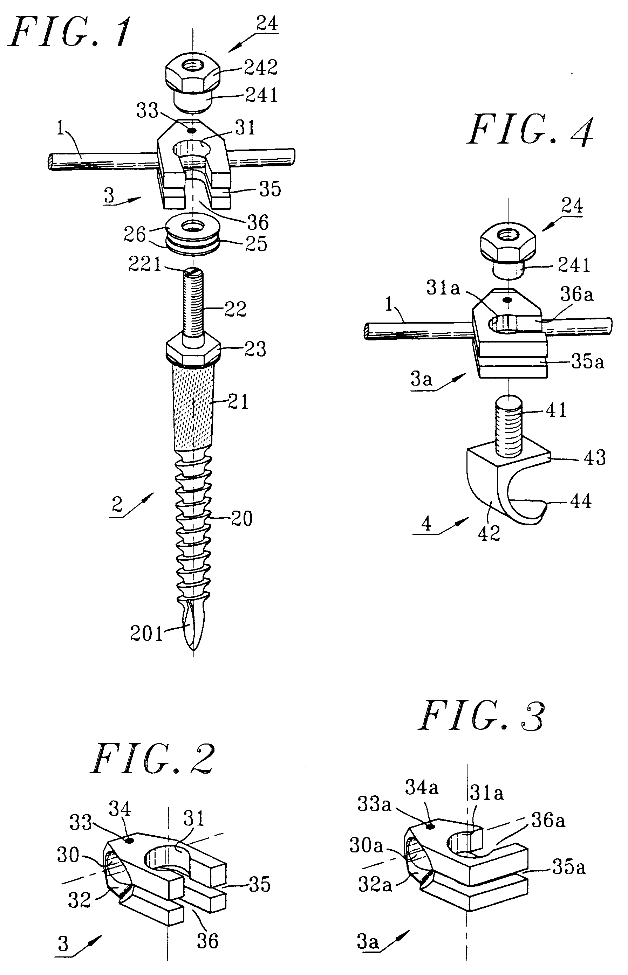

Un dispositif d'ostéosynthèse selon l'invention comprend au moins un assemblage de base pour relier une tige de consolidation 1 à un élément à tronçon fileté, qui est constituée par une vis pédiculaire 2 selon la figure 1, par l'intermédiaire d'un moyen de liaison rapide 3. Ces divers éléments et d'autres cités par la suite sont essentiellement métalliques, par exemple en un alliage à base de titane.

La **vis pédiculaire** 2 est en forme de goujon et est composée d'une partie partiellement filetée 20-21 sensiblement tronconique, d'un tronçon cylindrique 22 fileté sensiblement sur toute sa longueur, et d'une tête de préhension intermédiaire 23, ici à section hexagonale, située entre les partie et tronçon filetés.

La partie 20-21 comprend, sensiblement sur la moitié de sa longueur, un tronçon sensiblement tronconique 20 ayant un pas de filetage rond relativement large, terminé par une extrémité autotaraudeuse à pointe mousse 201, c'est-à-dire une pointe comportant deux rainures axiales opposées avec une section en vé, de manière à pénétrer facilement et être ancré intimement dans l'os spongieux d'un corps vertébral. Un tronçon tronconique 21 disposé entre le tronçon 20 et la tête 23 complète la partie de vis 20-21. Le tronçon 21 est recouvert d'un revêtement rugueux en titane pur poreux afin que ses micro-anfractuosités soient comblées par croissance osseuse du pédicule vertébral où il est implanté. Cet ancrage du tronçon rugueux 21 dans le pédicule assure une stabilité primaire au dispositif, contrairement à une liaison fibreuse avec un tronçon lisse de vis connue en alliage de Nickel-Chrome ou Cobalt-Chrome traversant le pédicule. La tête de préhension 23 est destinée à être prise par une clé de manoeuvre correspondante pour visser le tronçon fileté 20 dans les pédicule et corps vertébral jusqu'à ce que la tête 23 bute contre l'os cortical.

Le second tronçon fileté 22 constitue l'extrémité postérieure extra-vertébrale de la vis 2 après implantation, et est destiné à coopérer avec le moyen de liaison 3 et à recevoir un écrou 24. Comme montré en 22a à la figure 6, ce tronçon fileté postérieur 22 est conçu initialement relativement long de manière à adapter sa longueur par sciage préopératoire. La face extrême du tronçon fileté 22 comporte une fente 221 pour recevoir un tournevis, comme illustré à la figure 1, ou bien une extrémité polygonale, par exemple hexagonale, pour coopérer avec une clé de manoeuvre de manière à empêcher la rotation éventuelle de la vis 2 lors du serrage du moyen de liaison 3 par l'écrou 24, comme on le verra par la suite.

Comme montré aux figures 1 et 2, un moyen de liaison rapide est une **attache** 3 se présentant sensiblement sous la forme d'un petit pavé qui est partagé en deux faces antérieure et postérieure symétriques par rapport à une fente 35. Des premières moitiés, ici biseautées trapézoïdalement, des faces antérieure et postérieure de l'attache 3, sensiblement "verticales" après implantation du dispositif, sont percées parallèlement en un alésage lisse 30 recevant à coulissement la tige 1. Des secondes moitiés des deux faces antérieure et postérieure de l'attache 3 sont percées perpendiculairement par un second alésage lisse 31 qui est perpendiculaire au premier alésage 30 et non concourant avec ce dernier. L'alésage 31 a une section contenue dans les sections polygonales de la tête 242 de l'écrou 24 et de la tête 23 de la vis 2. Le tronçon fileté 22 d'une vis 2 notamment est reçu librement dans l'alésage 31.

Les extrémités du premier alésage 30 offrent des fraisures 32 facilitant la pénétration de la tige 1 dans l'alésage 30. Sur la face postérieure de l'attache 3 est prévu un petit trou taraudé 33 débouchant radialement dans le premier alésage lisse 30 afin qu'une petite vis de pression sans tête à six pans creux 34 vissée dans le trou 33 maintienne immobile l'attache 3 sur la tige 1 à une position temporaire souhaitée.

L'attache 3 comporte également deux fentes rectangulaires 35 et 36 qui s'étendent sensiblement perpendiculairement l'une à l'autre à partir d'une face "latérale" de l'attache qui est parallèle à l'alésage 30 et opposée à l'alésage 30 par rapport à l'alésage 31 et qui est orientée sensiblement vers la gauche ou droite après implantation du dispositif (figure 6). La première fente 35 est pratiquée suivant un plan sensiblement médian de l'attache coplanaire à l'axe de l'alésage 30. La fente 35 est traversée perpendiculairement par l'alésage 31, débouche axialement dans l'alésage 30, et a une largeur très nettement inférieure au diamètre de la tige 1 et de l'alésage 30. La seconde fente 36 débouche axialement dans le second alésage 31, est sensiblement perpendiculaire au premier alésage 30, et offre une largeur supérieure au diamètre du tronçon fileté de vis 22 et inférieure au diamètre de l'alésage 31. Le second alésage 31 reçoit une jupe complémentaire cylindrique à extrémité chanfreinée 241 qui est disposée sous la tête polygonale 242 de l'écrou 24. La jupe 241 est plus courte que l'alésage 31 et a un diamètre sensiblement égal au diamètre de l'alésage 31 et plus grand que la largeur de la fente 36 afin que la jupe ne s'échappe pas de l'attache après serrage de l'écrou 24.

La réalisation illustrée à la figure 1 évite avantageusement un glissement latéral de l'attache 3 par rapport à la vis 2 lors de l'assemblage de ces derniers, grâce à l'emprisonnement de la jupe 241 dans l'alésage 31 qui est plus large que la fente 36, bien que le tronçon fileté 22 passe à travers la fente 35 pendant l'assemblage du dispositif.

Ainsi il apparaît que l'attache 3 constitue un collier serrant la tige 1 et de liaison de la tige 1 à la vis 2. La tige coulisse dans l'alésage 30 lors de l'assemblage et positionnement de différentes pièces du dispositif d'ostéosynthèse et après implantation de la vis 2. Les faces antérieure et postérieure de l'attache 3 sont enserrées entre la tête de vis 23 et la tête d'écrou 242 de manière à fléchir sensiblement lesdites faces l'une vers l'autre et rétrécir la première fente 35 pour bloquer par pinçage la tige 1, et simultanément lier rapidement et solidement la vis 1 à l'attache 3 et donc à la tige 1. Grâce à la faible largeur de la première fente 35 de l'attache 3, la quasi-totalité de la périphérie circulaire du tronçon de la tige 1 contenue dans l'alésage 30 est pincée dans le premier alésage 30 qui, initialement fendu, se referme et cintre la tige 1. Ce pinçage quasi-total empêche tout glissement de l'attache 3 le long et/ou autour de la tige 1, et ainsi confère une surface porteuse davantage étendue et une liaison davantage rigide entre la tige et la vis 2 comparativement à la technique antérieure. Lors du vissage de l'écrou 24 et du rétrécissement de la fente 35, la vis 2 est maintenue immobile, le cas échéant, par la tête 23 ou la fente 221.

En outre, un relâchement du pinçage de la tige 1 par l'alésage 30 pour modifier les positions relatives de la vis 2 et de la tige 1 est obtenu en dévissant faiblement l'écrou 24, tout en maintenant encore la jupe 241 dans l'alésage 31 afin que le tronçon fileté 22 ne s'échappe pas par la fente 36.

Selon une autre variante montrée à la figure 3, une **attache de liaison 3a** présente, d'une manière analogue à l'attache 3 à la figure 2, un premier alésage lisse 30a dans lequel débouche une première fente 35a axiale à cet alésage, un second alésage lisse 31a perpendiculaire à l'alésage 30a, et un petit trou taraudé 33a pour vis de serrage 34a, radial à l'alésage 30. Dans l'attache 3a est ménagée une seconde fente 36a qui, comme la fente 36 dans la première attache 3, débouche axialement dans le second alésage 31 et est perpendiculaire à la première fente 35a, mais qui est pratiquée latéralement dans une face "supérieure" ou "inférieure" de l'attache 3a sensiblement parallèlement, et non perpendiculairement, au premier alésage 30a.

L'invention prévoit ainsi deux types d'attache à fente latérale 36a, selon que la fente 36a est orientée vers le "haut", ou vers le "bas" en direction du coccyx, comme montré à la figure 6.

Ces trois types d'attache, en conjugaison avec le montage à coulissement et rotation de l'alésage 30, 30a sur la tige 1, permettent ainsi d'aborder le tronçon fileté 22 d'une vis 3 (ou celui 41 d'une griffe vertébrale 4; figure 4) quasiment par n'importe quelle direction. En particulier, l'une choisie de ces attaches peut relier une tige 1 à une vis, quelle que soit l'orientation de la tige par rapport à la vis, dans un plan sensiblement perpendiculaire à la vis. Les petites vis de serrage temporaire 34, 34a assistent au maintien des attaches correspondantes 3, 3a à des positions souhaitées le long d'une tige commune 1 lorsque plusieurs attaches 3, 3a montées sur la tige 1 doivent être engagées par les fentes 36, 36a et les alésages 31, 31a simultanément dans des tronçons filetés 22 de vis correspondantes 2 (ou tronçons filetés 41 de griffes 4; figure 4).

L'assemblage entre une tige 1, un élément à tronçon fileté telle qu'une vis 2, et une attache 3, 3a comprend, en outre de préférence, un **moyen** traversé par le tronçon fileté 22 de la vis 2 et disposé entre la tête de vis 23 et la tête d'écrou 242 **pour amortir** des micro-déplacements relatifs entre la tige 1 et la vis 2. Ce moyen pour amortir contribue ainsi à annuler des micro-déplacements rachidiens d'une vertèbre à l'autre et donc d'une vis pédiculaire à l'autre et ainsi à éviter des fêlures ou fractures de la vis 2 et/ou de la tige 1. Ce moyen pour amortir comprend une rondelle 25 en matériau amortissant, tel qu'élastomère ou polyuréthanne ou silicone, enfilé sur le tronçon fileté 22. Selon la réalisation illustrée à la figure 1, cette rondelle 25 est entretoisée par deux rondelles de guidage métalliques 26, qui sont de préférence termocollées à la rondelle 25; cet ensemble de rondelles 25 + 26 est enfilé autour du tronçon 22 et est enserré à la base du tronçon 22, entre la tête de vis 23 et la face antérieure de l'attache 3, ou 3a.

Selon d'autres variantes, ce premier assemblage de rondelles ne peut comporter qu'une rondelle 25 en matériau amortissant et une seule rondelle métallique 26. Un second assemblage de rondelles comportant au moins une rondelle en matériau amortissant soit remplace le premier assemblage de rondelles, soit est combiné avec celui-ci, et est disposé entre la face postérieure de l'attache 3, 3a et la tête 242 de l'écrou 24.

En référence à la figure 4, l'élément à tronçon fileté constitué précédemment par une vis 2 est ici sous la forme d'une **première griffe vertébrale** 4. Cette griffe 4 comporte également un tronçon fileté cylindrique 41 destiné à recevoir un écrou 24 et à pénétrer dans un second alésage 31, 31a d'attache 3, 3a à travers la seconde fente 36, 36a de cette attache; à titre d'exemple, la figure 4 montre un assemblage de griffe 4 avec une attache 3a à seconde fente latérale 36a. Sous-jacente au tronçon fileté 41 de la griffe 4 est prévue une partie en forme de demi-croissant sensiblement circulaire 42, formant une "griffe", qui est séparée du tronçon fileté 41 par une tête rectangulaire 43 et qui est terminée par une extrémité sensiblement pointue 44. La partie 42 est destinée à accrocher des bordures de divers évidemments des vertèbres notamment pédiculaires, lombaires, thoraciques, sus-laminaires, sous-laminaires, et transversaires. Comme pour la vis 2, la griffe 4 peut être abordé sensiblement latéralement, par la droite ou la gauche, ou par le haut ou le bas, par l'une des trois attaches 3, 3a, et sa partie en forme de demi-croissant 42 peut-être orientée selon une direction quelconque grâce à la rotation du tronçon fileté 41 dans l'alésage 31, 31a de l'attache 3, 3a.

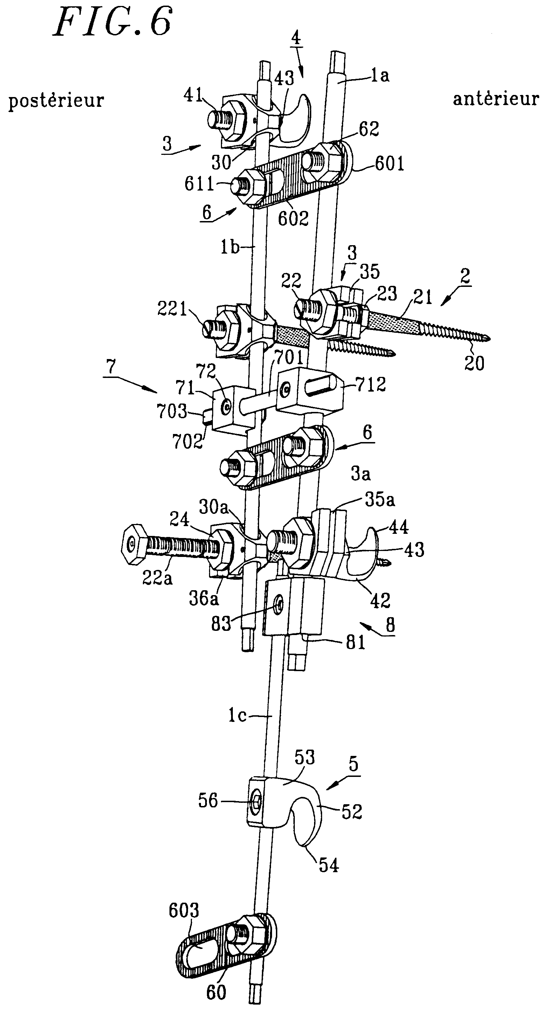

Une **seconde griffe** 5 analogue à la griffe précédente 4 est montrée à la figure 5. Cette seconde griffe 5 comporte une partie en forme de demi-croissant 52 analogue à la partie 42, et une tête parallélépipédique 53 comportant un alésage 55 qui est monté à coulissement sur une tige de consolidation 1. Selon la réalisation illustrée à la figure 5, la pointe 54 de la griffe est située dans un plan axial à l'alésage 55; toutefois, selon d'autres réalisations, la partie en forme de demi-croissant 52 peut être orientée d'une manière quelconque et en fonction des besoins autour d'un axe médian de la tête 53 perpendiculaire à l'alésage 55. Le long de cet axe médian est ménagé un trou taraudé 51 à partir de la face souvent postérieure de la tête 53 opposée à la partie en forme de demi-croissant 52 et débouchant radialement dans l'alésage 55. Une petite vis de pression 56 sans tête à six pans creux pénètre dans le trou 51 de manière à bloquer en translation et en rotation la tige 1 dans l'alésage 55, et ainsi maintenir la griffe 5 sur la tige 1. Une telle seconde griffe 5 montée à coulissement sur la tige 1 peut être ancrée par exemple dans une apophyse transverse ou épineuse d'une vertèbre.

La figure 6 montre schématiquement en vue latéro-postérieure, à titre d'exemple, un ensemble rigide de diverses pièces pouvant entrer dans la constitution d'un dispositif d'ostéosynthèse pour consolidation rachidienne selon l'invention. Dans ce dispositif on y retrouve des vis pédiculaires 2 avec des écrou 24 et moyen amortissant 25 + 26 ainsi que des premières griffes 4 avec écrou 24 qui sont reliées à plusieurs tiges 1, ici au nombre de trois 1a, 1b et 1c, par l'intermédiaire d'attaches correspondantes 3 et 3a ainsi qu'une seconde griffe vertébrale 5 fixée autour de la tige 1c.

Les première et seconde tiges 1a et 1b sont deux tiges à relier à des ensembles de vis respectivement situés à droite et à gauche de l'apophyse épineuse du rachis et sont donc disposées sensiblement côte à côte grâce à au moins l'un de deux moyens transversaux de stabilisation 6 et 7.

Les première et troisième tiges 1a et 1c sont disposées le long du même coté de l'apophyse épineuse, par exemple le côté droit. La troisième tige 1c peut être destinée à prolonger une consolidation rachidienne déjà effectuée par les moyens liés à la première tige 1a; un moyen longitudinal de stabilisation 8 est donc prévu entre des extrémités des tiges 1a et 1c.

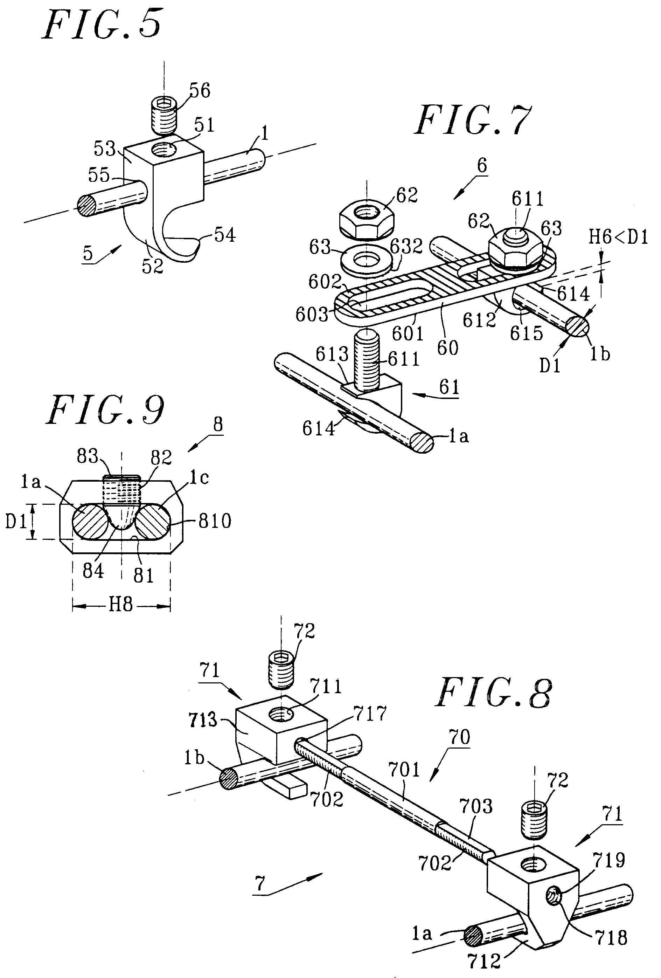

Le **premier moyen transversal de stabilisation** 6 est montré à la figure 7 et comprend une plaque de stabilisation sensiblement rectangulaire allongée 60, deux premiers crochets de tige 61 ainsi que deux écrous 62 et deux rondelles crantées 63.

La plaque 60 présente une face antérieure lisse 601 à appliquer sur les deux tiges 1a et 1b et une face postérieure 602 ayant des petites crénelures parallèles propres à engrener avec des faces crantées 632 des deux rondelles 63. Deux lumières oblongues 603 sont ménagées longitudinalement dans la plaque 60 pour être disposée sensiblement transversalement aux tiges 1a et 1b. Selon une autre variante, les deux lumières sont remplacées par une lumière plus longue.

Les crochets 61 ont une forme analogue au premières griffes vertébrales 4, tel que celle montrée à la figure 4, mais ont une taille réduite sensiblement de moitié comparativement aux griffes 4. Ainsi un crochet 61 comporte un tronçon fileté 611 et une partie de prise en forme de demi-croissant circulaire 612 ainsi qu'une tête rectangulaire 613 entre le tronçon 611 et la partie 612.

D'une part, la longueur de la tête de crochet 613 est sensiblement inférieure à celle des lumières 603 dans la plaque 60. D'autre part, la surface concave sensiblement semi-circulaire 615 du crochet 61 a un diamètre sensiblement supérieur au diamètre D1 des tiges 1a, 1b et présente une ouverture latérale 614 dont les surfaces parallèles sont sensiblement inclinées par rapport à l'axe du tronçon fileté 611 et vers le tronçon 611, et donc par rapport à la plaque 60 après serrage. Ces deux conditions permettent à une tige 1a, 1b logée dans le fond de la surface concave 615 du crochet d'être poussée par une plaque 60 dont une lumière encadre largement la tête 613, sans que la tige 1a, 1b, ne puisse s'échapper du crochet 61. La distance H6 entre la surface lisse 601 de la plaque et la pointe 614 du crochet est alors inférieure au diamètre D1 de la tige, comme montré à droite dans la figure 7.

Cette liaison rigide entre la partie en forme de demi-croissant 612 d'un crochet 61 et une plaque 60 est obtenue :
(a) en crochetant la tige correspondante 1a, 1b par la surface concave 615,
(b) en introduisant à coulissement la tête de crochet 613 dans une lumière 603 de la plaque,
(c) en enfilant une rondelle crantée 602 sur le tronçon fileté 611 saillant de la face crénelée 602, au-dessus de la lumière 603,
(d) en engrenant les crans de la rondelle crantée 63 dans les crénelures de la face 602, et
(e) finalement en vissant l'écrou 62 sur le tronçon 611 de manière à ce que l'écrou avec la surface concave de crochet 615 ensert la rondelle 63, la plaque 60 au niveau de la lumière 603, et la tige correspondante 1a, 1b.

Deux premiers crochets 61 sont ainsi montés sur deux tiges 1a et 1b et reliés par une plaque commune 60. Les crochets peuvent être disposés dos-à-dos entre les tiges 1a et 1b comme montré à la figure 7, ou bien face-à-face à l'extérieur de l'ensemble des deux tiges, ou bien encore en série et respectivement à l'extérieur et à l'intérieur de l'ensemble des deux tiges.

Les deux tiges 1a et 1b peuvent être stabilisées par deux plaques 60 et quatre crochets 61 en formant un quadrilatère indéformable de manière à consolider l'assemblage de ces pièces en contraction ou en décontraction, comme montré à la figure 6. A cet égard, l'écartement à des extrémités des tiges 1a et 1b peut être augmenté ou diminué, après desserrage des écrous 62 de la plaque de stabilisation 60 et dégagement des rondelles crantées 63 de la face de plaque 602, en éloignant ou rapprochant les crochets de tige et rondelles crantées correspondants 61 et 63 par coulissement des crochets dans les lumières 603 de la plaque 60.

Un **second moyen transversal de stabilisation** 7 comprend, en référence à la figure 8, une barre mince 70 ainsi que deux seconds crochets de tige 71 munis chacun d'une petite vis de pression 72 sans tête et à six pans creux.

La barre 70 comporte une partie centrale cylindrique lisse 701 séparant deux parties extrêmes filetées 702 qui offrent des méplats longitudinaux coplanaires 703 parallèles à l'axe de la barre 70.

Chacun des seconds crochets de tige 71 a une forme analogue à une seconde griffe vertébrale 5, telle que celle montrée à la figure 5, mais ayant une taille réduite sensiblement de moitié. Ainsi un crochet 71 comporte une tête parallélépipédique 713 et une partie de prise sensiblement en forme de demi-croissant circulaire 712 pour crocheter une tige 1a, 1b. La tête 713 comporte un trou demi-taraudé oblong 717 qui s'étend parallèlement à la partie de prise en crochet 712 et perpendiculairement à la tige 1a, 1b crocheté par celle-ci. Perpendiculairement à la face postérieure de la tête 713 et dans le prolongement axial d'une section oblongue du trou 717 est pratiquée un trou taraudé 711 pour recevoir une vis de pression 72 perpendiculaire à la fois à la barre 70 et à la tige 1a, 1b après assemblage.

Le trou 717 est partagé, en section transversale, en une première portion longitudinale concave au moins partiellement taraudée 718 et une seconde portion longitudinale concave lisse 719 par rapport à un plan parallèle à l'axe du trou 717 et perpendiculaire au trou 711. La section oblongue du trou 718 est plus longue et au moins sensiblement aussi large que le diamètre des parties filetées de barre 702. La première portion 718 est de préférence taraudée au moins au fond avec un filetage correspondant aux parties filetées 702 de la barre 70 et est disposée à l'opposé du trou taraudé 711 par rapport à l'axe du trou 717, vers la partie antérieure en forme de demi-croissant 712. La seconde portion lisse 719 est disposée vers la face postérieure du trou 717, et le trou taraudé 711 débouche transversalement dans cette portion lisse.

Le second moyen de stabilisation 7 est monté de la manière suivante.

Chacun des seconds crochets 71 dont les trous oblongs demi-taraudés 717 ont été dégagés des vis 72, sont enfilés sur les parties extrêmes filetées 702 de la barre 70 de part et d'autre des deux tiges 1a et 1b et sont disposés avec leurs parties 712 en forme de demi-croissant circulaire, par exemple en regard l'une de l'autre comme montré à la figure 8. Après crochetage des tiges 1a et 1b par les parties de prise 712, pour tenter de les rapprocher selon la figure 8, ou de les éloigner selon d'autres variantes déjà évoquées pour les crochets 61, les parties filetées de barre 702 sont engrenées dans les portions taraudées 718 des trous 717. Ces engrènements sont maintenus en vissant dans les trous 711 les vis 72 dont les bouts plats poussent les méplats 703 contre les portions taraudées 718. Dans ces conditions, la barre 70 est arrêtée aussi bien en rotation grâce à la pression des vis 72 exercée sur les méplats 703, qu'en translation axiale grâce à l'engrènement autobloquant du filetage des parties de barre 702 dans le taraudage des portions de trou demi-taraudé 718.

Cet assemblage est très résistant contre tout écartement ou éloignement des crochets 71 grâce au nombre relativement élevé des filets coopérant entre les portions de trou 718 et les parties de barre 702. En outre, cet assemblage est rapide à monter ou démonter, sans nécessiter un dévissage long des extrémités de barre 702, puisque celles-ci sont posées dans les trous oblongs 718.

Comme montré aux figures 6 et 9, le **moyen** **longitudinal de stabilisation** est sous la forme d'un raccord de tiges 8. Ce raccord 8 a une forme parallélépipédique dans laquelle est ménagée un orifice longitudinal 81 à section transversale oblongue. La largeur de l'orifice 81 et le diamètre des parois extrêmes semi-circulaires 810 de l'orifice 81 sont sensiblement égaux au diamètre D1 des tiges 1a, 1c. La face postérieure du raccord 8 est percée d'un trou taraudé 82 débouchant transversalement et centralement dans l'orifice oblong 81. Le trou 82 est propre à recevoir une petite vis-pointeau 83 sans tête à six pans creux ayant un bout pointu lisse tronconique 84.

La hauteur H8 (longueur transversale) de l'orifice 81 est supérieure à la somme des diamètres des tiges 1a et 1c, mais inférieure à la somme des diamètres des tiges 1a et 1c et de la vis-pointeau 83. Dans ces conditions, après avoir enfilé les extrémités des deux tiges 1a et 1c dans l'orifice oblong 81, la vis-pointeau 83 est vissée dans l'orifice 82 afin que son bout pointu tronconique 84 écarte progressivement les tiges 1a et 1c et les coince fermement contre les parois semi-circulaires en regard 810 du trou 81, ce qui relie définitivement et solidement les deux tiges 1a et 1c.

Selon d'autres variantes, un raccord présente un orifice 81 ayant une hauteur plus élevée pour y relier par coinçage plusieurs tiges côte à côte. Par exemple, ladite hauteur H8 est supérieure à la somme des diamètres de trois, respectivement quatre tiges de consolidation et inférieure à la somme des diamètres des trois respectivement quatre tiges et des diamètres de deux, respectivement trois vis-pointeau vissées transversalement dans le raccord et ayant chacune une extrémité tronconique disposée entre deux tiges respectives.

## Revendications

1. Dispositif d'ostéosynthèse comprenant une tige (1), un élément (2;4) ayant un tronçon fileté (22;41) s'étendant à partir d'une tête (23,43) et muni d'un écrou (24), et une attache de liaison (3), l'attache (3) comprenant un premier alésage (30) traversé par ladite tige (1), une première fente (35) débouchant axialement dans le premier alésage, et un second alésage (31) sensiblement perpendiculaire à la première fente (35) et traversé par une partie dudit tronçon fileté (22;41) localisée entre la tête d'élément et l'écrou,
caractérisé par
le caractère lisse du premier alésage (30), et
une seconde fente (36) perpendiculaire à la première fente (35), débouchant axialement dans le second alésage (31) et ayant une largeur supérieure au diamètre du tronçon fileté (22;41).

2. Dispositif conforme à la revendication 1, caractérisé en ce qu'il comprend une jupe cylindrique (241) de l'écrou (24) ayant un diamètre qui est sensiblement égal au diamètre du second alésage (31) et supérieur à la largeur de la seconde fente (36).

3. Dispositif conforme à la revendication 1 ou 2, dans lequel la seconde fente (36,36a) s'étend sensiblement perpendiculairement ou parallèlement au premier alésage (30;30a).

4. Dispositif conforme à l'une quelconque des revendications 1 à 3, dans lequel l'attache (3) comprend un moyen de pression (34) radial au premier alésage (30) pour y maintenir immobile temporairement l'attache (3) sur la tige (1).

5. Dispositif conforme à l'une quelconque des revendications 1 à 4, comprenant un moyen (25,26) traversé par le tronçon fileté (22;41) et disposé entre la tête d'élément (23;43) et l'écrou (24) pour amortir des déplacements relatifs entre la tige (1) et ledit élément (2;4).

6. Dispositif conforme à la revendication 5, dans lequel le moyen pour amortir comprend une rondelle en matériau amortissant (25) qui est de préférence en contact avec une ou deux rondelles métalliques (26).

7. Dispositif conforme à l'une quelconque des revendications 1 à 6, dans lequel ledit élément (2) comporte une partie (20-21) au moins partiellement filetée et séparée dudit tronçon fileté (22) par la tête (23).

8. Dispositif conforme à l'une quelconque des revendications 1 à 6, dans lequel ledit élément (4) comporte une partie en forme de griffe (42) séparée dudit tronçon fileté (41) par la tête (43).

9. Dispositif conforme à l'une quelconque des revendications 1 à 8, comprenant un moyen (5) ayant un alésage traversé à coulissement par la tige (1) et ayant une partie en forme de griffe (52), et un moyen de pression (56) radial à la tige pour maintenir immobile ledit moyen traversé à coulissement (5) par la tige (1).

10. Dispositif conforme à l'une quelconque des revendications 1 à 9, comprenant une seconde tige (1b) disposée latéralement à la première tige précitée (1a), et un premier moyen transversal de stabilisation (6) reliant les première et seconde tiges.

11. Dispositif conforme à l'une quelconque des revendications 1 à 9, comprenant
une seconde tige (1b) disposée latéralement à la première tige précitée (1a),
une plaque (60) transversale aux tiges,
deux crochets (61) ayant chacun un tronçon fileté (611) traversant une lumière (6O3) de la plaque et une partie de prise (612) entourant partiellement l'une respectif des première et seconde tiges (1a, 1b), et
deux écrous (62) vissés respectivement autour des tronçons filetés de crochet (611) pour serrer la plaque (6O) contre les première et seconde tiges (1a, 1b) afin d'emprisonner les tiges entre la plaque et les parties de prise de crochet (612).

12. Dispositif conforme à la revendication 11, dans lequel la partie de prise (612) de chaque crochet (61) est situé dans la lumière (6O3) et a une ouverture (614) sensiblement inclinée par rapport au et vers le tronçon fileté (611) dudit crochet.

13. Dispositif conforme à la revendication 11 ou 12, dans lequel l'une (6O2) de faces de la plaque (6O) est crénelée pour coopérer avec des rondelles crantées (63) enfilées autour des tronçons filetés (611) des crochets (61).

14. Dispositif conforme à l'une quelconque des revendications 1 à 9, comprenant
une seconde tige (1b) disposée latéralement à la première tige précitée (1a),
une barre (7O) transversale aux tiges,
deux crochets (71) ayant des parties de prise (712) à crocheter aux tiges, et
des moyens (717, 72) pour fixer rapidement les crochets à des extrémités (702, 7O3) de la barre.

15. Dispositif conforme à la revendication 14, dans lequel
les extrémités (702, 703) de la barre (70) comportent à la fois une partie filetée longitudinale (702) et un méplat longitudinal (703), et
les moyens pour fixer dans chacun des crochets (71) comprennent un trou (717) oblong pour recevoir librement l'une des extrémités de barre (702, 703),
ledit trou ayant une partie longitudinale taraudée (718) pour engrener avec la partie filetée de ladite une des extrémités de barre, et une partie lisse (719) dans laquelle débouche un trou taraudé (711) contenant une vis de pression (72) pressée sur le méplat (703) de ladite une des extrémités de barre.

16. Dispositif conforme à l'une quelconque des revendications 1 à 15, comprenant une troisième tige (1c) sensiblement parallèle à la première tige (1a), et un moyen de stabilisation longitudinal (8) reliant des extrémités des tiges.

17. Dispositif conforme à l'une quelconque des revendications 1 à 15, comprenant
une troisième tige (1c) sensiblement parallèle à la première tige (1a), et un raccord (8) ayant un orifice longitudinal (81) à section oblongue traversé par les première et troisième tiges, et
un moyen (83) pour écarter les première et troisième tiges afin de bloquer les première et troisième tiges dans ledit orifice (81).

18. Dispositif conforme à la revendication 17, dans lequel ledit moyen pour écarter comprend une vis (83) ayant un bout pointu (84) qui débouche transversalement dans ledit orifice (81) du raccord et entre les première et troisième tiges (1a, 1c) et coince les première et troisième tiges contre des parois en regard (810) du trou (81).

19. Dispositif conforme à l'une quelconque des revendications 1 à 15, comprenant plusieurs tiges (1a, 1b, 1c), un raccord (8) ayant un orifice longitudinal (81) à section oblongue traversé par les tiges, et plusieurs moyens (83) en nombre inférieur d'une unité aux tiges pour chacun écarter deux tiges respectives afin de les bloquer dans ledit orifice.

## Claims

1. An osteosynthesis device comprising a rod (1), a member (2;4) having a threaded section (22;41) extending from a head (23;43) and fitted with a nut (24), and a connecting fastener (3), the fastener (3) comprising a first bore (30) passed through by said rod (1), a first slit (35) opening axially into the first bore, and a second bore (31) substantially perpendicular to the first slit (35) and passed through by a portion of said threaded section (22;41) located between the member head and the nut,
characterized by
the smooth character of the first bore (30), and
a second slit (36) perpendicularly to the first slit (35), opening axially into the second bore (31) and having a width greater than the diameter of said threaded member section (22;41).

2. Device according to the claim 1, characterized in that it comprises a cylindrical shank (241) of the nut (24) having a diameter that is substantially equal to the diameter of the second bore (31) and more than the width of the second slit (36).

3. Device according to the claim 1 or 2, characterized in that the second slit (36;36a) extends substantially perpendicular to the first bore (30;30a).

4. Device according to any one of claims 1 to 3, characterized in that the fastener (3) comprises a locking means (34) radial to the first bore (30) in order to temporarily maintain the fastener (3) motionless on the rod (1).

5. Device according to any one of claims 1 to 4, comprising a means (25;26) passed through by the threaded section (22;41) and provided between the member head (23;43) and the nut (24) for damping relative movements between the rod (1) and said member (2;4).

6. Device according to the claim 5, characterized in that the damping means comprises a washer made of damping material (25), which is preferably in contact with one or two metal washers (26).

7. Device according to any one of claims 1 to 6, characterized in that said member (2) comprises a portion (20-21) which is at least partially threaded and separated from said threaded section (22) by the head (23).

8. Device according to any one of claims 1 to 6, characterized in that said member (4) comprises a claw-shaped portion (42) separated from said threaded section (41) by the head (43).

9. Device according to any one of claims 1 to 8, comprising a means (5) having a bore slidably passed through by the rod (1) and having a claw-shaped portion (52), and a locking means (56) radial to the rod for maintaining motionless said means slidably passed through (5) by the rod (1).

10. Device according to any one of claims 1 to 9, comprising a second rod (1b) disposed laterally to the previously mentioned first rod (1a), and a first transversal stabilizing means (6) connecting the first and second rods.

11. Device according to any one of claims 1 to 9, comprising
a second rod (1b) disposed laterally to the previously mentioned first rod (1a),
a plate (60) transversal to the rods,
two hooks (61) each having a threaded section (611) passing through a slot (603) of the plate and a holding portion (612) partially surrounding respective one of first and second rods (1a, 1b), and two nuts (62) respectively screwed onto threaded hook sections (611) for tightening the plate (60) against the first and second rods (1a, 1b) thereby confining the rods between the plate and the hook holding portions (612).

12. Device according to the claim 11, characterized in that the holding portion (612) of each hook (61) is located into the slot (603) and has an opening (614) substantially inclined in relation to and towards the threaded section (611) of said hook.

13. Device according to the claim 11 or 12, characterized in that one (602) of sides of the plate (60) is indented thereby cooperating with notched washers (63) slid over the threaded sections (611) of the hooks (61).

14. Device according to any one of the claims 1 to 9, comprising :
a second rod (1b), disposed laterally to the previously mentioned first rod (1a),
a bar (70) transversal to the rods,
two hooks (71) having hooking portions (712) to be hooked to the rods, and
means (717, 72) for quickly securing the hooks to ends (702, 703) of the bar.

15. Device according to the claim 14, characterized in that
the ends (702, 703) of the bar (70) each comprise a longitudinal threaded section (702) and a longitudinal flat surface (703), and
the securing means in each of the shooks (79) comprise an oblong hole (717) for free racommodating one of the bar ends (702,703),
said hole having a tapped longitudinal portion (718) for meshing with the tapped portion of said one of said bar ends, and a smooth portion (719) into which opens a tapped hole (711) containing a locking screw (72) pressed against said flat surface (703) of said one of the bar ends.

16. Device according to any one of claims 1 to 15, comprising a third rod (1c) substantially parallel to the first rod (1a), and a longitudinal stabilizing means (8) connecting the ends of the rods.

17. Device acording to any one of claims 1 to 15, comprising
a third rod (1c) substantially parallel to the first rod (1a), and
a connector (8) having a longitudinal orifice (81) with an oblong cross-section passed through by the first and third rods, and
a means (83) for spacing apart the first and third rods thereby blocking the first and third rods in said orifice (81).

18. Device according to the claim 17, characterized in that said spacing means comprises a screw (83) having a pointed end (84) which opens transversely into said orifice (81) of the connector and between the first and third rods (1a, 1c) and wedges the first and third rods against opposite walls (810) of the hole (81).

19. Device according to any one of claims 1 to 15, comprising plural rods (1a, 1b, 1c), a connector (8) having a longitudinal orifice (81) with an oblong cross-section passed through by said rods, and plural means (83) fewer by one unit in number than the rods for each spacing apart respective two rods thereby blocking rods in said orifice.

## Patentansprüche

1. Osteosynthesevorrichtung mit einer Stange (1), mit einem Element (2; 4), das einen mit Gewinde versehenen Abschnitt (22; 41) aufweist, der sich von einem Kopf (23; 43) ausgehend erstreckt und mit einer Mutter (24) versehen ist, sowie mit einem Verbindungsstück (3), welches eine erste von der Stange durchsetzte Bohrung (30), wobei ein erster Schlitz (35) sich axial in die erste Bohrung öffnet, sowie eine zweite Bohrung (31) aufweist, die sich im wesentlichen senkrecht zu dem ersten Schlitz (35) erstreckt und von einem Teil des mit Gewinde versehenen Abschnittes (22; 41) quer durchsetzt ist, welcher zwischen dem Kopf des Elementes und der Mutter lokalisiert ist,
gekennzeichnet durch
eine Glättung der ersten Bohrung (30) sowie durch einen zweiten Schlitz (36), der sich senkrecht zum ersten Schlitz erstreckt und sich axial in die zweite Bohrung (31) öffnet und eine Weite hat, die größer ist, als der Durchmesser des mit Gewinde versehenen Abschnitts (22; 41).

2. Vorrichtung nach Anspruch 1, dadurch gekennzeichnet, daß eine zylindrische Schürze (241) der Mutter (24) einen Durchmesser hat, der im wesentlichen gleich dem Durchmesser der zweiten Bohrung (31) und größer ist, als die Weite des zweiten Schlitzes (36).

3. Vorrichtung nach Anspruch 1 oder 2, in welcher der zweite Schlitz (36, 36a) sich im wesentlichen senkrecht oder parallel zur ersten Bohrung (30; 30a) erstreckt.

4. Vorrichtung nach einem der Ansprüche 1 bis 3, in welcher das Verbindungsstück (3) Mittel (34) aufweist, die geeignet sind, radialen Druck bezüglich der ersten Bohrung (30) auszuüben, um dort vorübergehend das Verbindungsstück (3) auf der Stange (1) unbeweglich zu halten.

5. Vorrichtung nach einem der Ansprüche 1 bis 4, bei der von dem mit Gewinde versehenen Abschnitt (22; 41) durchsetzte Mittel (25, 26) vorgesehen sind, die zwischen dem Kopf des Elementes (23; 43) und der Mutter (24) angeordnet sind, um relative Verlagerungen zwischen der Stange (1) und dem Element (2; 4) abzuschwächen.

6. Vorrichtung nach Anspruch 5, dadurch gekennzeichnet, daß die Mittel zum Abschwachen eine aus abschwächendem Werkstoff bestehende Scheibe (25) aufweisen, die vorzugsweise mit einer oder zwei metallischen Scheiben (26) in Kontakt steht.

7. Vorrichtung nach einem der Ansprüche 1 bis 6, in der das Element (2) einen Teil (20-21) aufweist, der wenigstens teilweise mit Gewinde versehen und durch den Kopf (23) von dem mit Gewinde versehenen Abschnitt (22) getrennt ist.

8. Vorrichtung nach einem der Ansprüche 1 bis 6, in welcher das Element (4) einen Teil in Form einer Klaue (42) aufweist, die von dem mit Gewinde versehenen Abschnitt (41) durch den Kopf (43) getrennt ist.

9. Vorrichtung nach einem der Ansprüche 1 bis 8 mit Mitteln (5), die eine Bohrung, die von der Stange (1) gleitend durchsetzt ist, sowie einen die Form einer Klaue (52) besitztenden Teil aufweisen, sowie mit Mitteln (56), die geeignet sind, radialen Druck auf die Stange auszuüben, um die Mittel (5), die von der Stange (1) gleitend durchsetzt sind, unbeweglich zu halten.

10. Vorrichtung nach einem der Ansprüche 1 bis 9 mit einer zweiten Stange (1b), die seitlich zu der ersten Stange (1a) angeordnet ist, sowie mit ersten transversal angeordneten Stabilisationsmitteln (6), die die erste und die zweite Stange verbinden.

11. Vorrichtung nach einem der Ansprüche 1 bis 9 mit einer zweiten Stange (1b), die seitlich zu der ersten Stange (1a) angeordnet ist,
mit einer sich quer zu den Stangen erstreckende Platte (60),
mit zwei Haken (61), von denen jeder einen mit Gewinde versehenen, ein Loch (603) der Platte durchsetzenden Abschnitt (611) und ein Greifteil (612) aufweist, der jeweils eine der ersten oder zweiten Stangen (1a, 1b) partiell umgreift, sowie mit zwei Muttern (62), die jeweils auf die mit Gewinde versehenen Abschnitte der Haken (611) aufgeschraubt sind, um die Scheibe (60) an der ersten bzw. zweiten Stange (1a, 1b) festzulegen, und um die Stangen zwischen der Platte und den Greifteilen der Haken (612) einzufangen.

12. Vorrichtung nach Anspruch 11, in welcher das Greifteil (612) jedes Hakens (61) in einem Loch (603) angeordnet ist und eine Öffnung (614) aufweist, die bezüglich und gegen den mit Gewinde versehenen Abschnitt (611) des Hakens geneigt ist.

13. Vorrichtung nach einem der Ansprüche 11 oder 12, in welcher eine (602) der Oberflächen der Platte (60) gerippt ist, um mit den gerasteten Scheiben zusammenzuwirken, die um die mit Gewinde versehenen Abschnitten (612) der Haken (61) aufgereiht sind.

14. Vorrichtung nach einem der Ansprüche 1 bis 9 mit einer zweiten Stange (1b), die seitlich zur ersten Stange (1a) angeordnet ist,
mit einem quer zu den Stangen angeordneten Riegel (70),
mit zwei Haken (71), die Greifteile (712) zum Ergreifen der Stangen besitzen, und mit Mitteln (717, 72) zum Schnell-Fixieren der Haken an den Enden (702, 703) des Riegels.

15. Vorrichtung nach Anspruch 14, bei der
die Enden (702, 703) des Riegels (70) zugleich einen longitudinalen mit Gewinde versehenen Teil (702) und eine longitudinale Abflachung (703) aufweisen und
wobei die Mittel zum Festlegen jedes der Haken (719 ein Langloch (717) zur freien Aufnahme eines der Enden des Riegels (702, 703) aufweisen,
wobei das Loch einen longitudinalen, mit Gewinde versehenen Teil (718) zum Eingriff mit dem mit Gewinde versehenen Teil eines der Enden des Riegels, sowie einen geglätteten Teil (719) aufweist, in welchen das mit Innengewinde versehene Loch öffnet, wobei eine Druckschraube (72) auf die Abflachung (703) eines der Extremitäten des Riegels drückt.

16. Vorrichtung nach einem der Ansprüche 1 bis 15 mit einer dritten Stange (1c), die im wesentlichen parallel zur ersten Stange (1a) verläuft, sowie durch longitudinale Stabilisationsmittel (8), die die Enden der Stange verbinden.

17. Vorrichtung nach einem der Ansprüche 1 bis 15, mit einer dritten Stange (1c), die im wesentlichen parallel zur ersten Stange (1a) angeordnet ist, und mit
einem Übergangsstück (8) mit einer Längsöffnung (81) mit abgeflachtem Querschnitt, die von den ersten und dritten Stangen durchsetzt ist, und mit Mitteln (83) zum Spreizen der ersten und dritten Stangen, um die ersten und dritten Stangen in der Öffnung (81) zu fixieren.

18. Vorrichtung nach Anspruch 17, in welcher die Mittel zum Spreizen eine mit Spitze (84) versehene Schraube (83) aufweisen, die quer in die Öffnung (81) des Übergangsstücks und zwischen der ersten und dritten Stange (1a, 1c) mündet und die erste und dritte Stange gegen die Wände (810) des Loches (81) verkeilt.

19. Vorrichtung nach einem der Ansprüche 1 bis 15 mit mehreren Stangen (1a, 1b, 1c), einem Übergangsstück (8) mit einer Längsöffnung (81) von abgeflachtem Querschnitt, durch welche die Stangen sich erstrecken, sowie mit mehreren Mitteln (81), deren Anzahl um eine Einheit kleiner ist als die der Stangen, um zwei Stangen jeweils zu verkeilen und sie somit in der Öffnung zu blockieren.
